(19) Europäisches Patentamt
European Patent Office
Office européen des brevets

(11) Publication number: **0 094 498**
**A2**

(12) # EUROPEAN PATENT APPLICATION

(21) Application number: 83103048.1

(22) Date of filing: 28.03.83

(51) Int. Cl.[3]: **C 07 D 241/20**
C 07 D 241/44, C 07 D 213/38
C 07 D 215/38, C 07 D 277/42
C 07 D 401/14, C 07 D 295/18
A 61 K 31/495

(30) Priority: 06.05.82 US 375526
06.05.82 US 375507

(43) Date of publication of application: 23.11.83 Bulletin 83/47

(84) Designated Contracting States: AT BE CH DE FR GB IT LI NL SE

(71) Applicant: AMERICAN CYANAMID COMPANY
1937 West Main Street P.O. Box 60
Stamford Connecticut 06904(US)

(72) Inventor: Di Tu, Mila
17 Heather Hill Lane
Suffern New York 10901(US)

(72) Inventor: Shepherd, Robert Gordon
32 Bayberry Lane
Selbyville Delaware 19975(US)

(74) Representative: Wächtershäuser, Günter, Dr.
Tal 29
D-8000 München 2(DE)

(54) **Antiatherosclerotic 1-piperazine derivatives.**

(57) This disclosure describes novel 1-piperazinecarbonylcarboxamides and 1-piperazinethiocarboxamides, such as 4'-chloro-4-(2-pyrazinyl)-1-piperazinecarboxanilide and 4'-cyano-4-(2-pyrazinyl)-1-piperazinethiocarboxanilide, pharmaeutical compositions containing same, a process for their preparation, and a method of treating atherosclerosis therewith.

EP 0 094 498 A2

Croydon Printing Company Ltd.

28,864

# ANTIATHEROSCLEROTIC 1-PIPERAZINE DERIVATIVES

This invention is concerned with organic compounds which are useful as pharmaceutical agents. The compounds of the present invention are piperazines which are capable of ameliorating atherosclerosis in mammals. This invention further relates to novel piperazines and methods for the chemical synthesis of these compounds. The invention also relates to pharmaceutical compositions for the utilization of these compounds in the treatment of disease in mammals. The invention contemplates methods for treating atherosclerosis in a mammal in a manner designed to prevent, arrest, or reverse the course of the disease.

Specific compounds of this invention are 4-heteroaryl-1-piperazinecarbonyl, 4-aryl-1-piperazinecarbonyl, and 4-heteroarylmethyl-1-piperazinecarbonyl compounds, and this invention contemplates as novel compounds per se only certain types of 1-piperazinecarbonyl compounds which are not known in the art. The following references disclose compounds which are structurally related to the new compounds of this invention but which are sufficiently different in chemical structure to be considered patentably distinct: Chemical Abstract 28:13481; 31:19492; 32:5705; 33:28975; 42:1942f; 48:13694; 56:10143e; 68:87274t; and 84:4904e. None of the compounds disclosed in these references are reported or claimed to be useful for the treatment of atherosclerosis in mammals.

Other specific compounds of this invention are 4-heteroaryl-1-piperazinethiocarboxamides, and this invention contemplates as novel compounds per se only certain

types of 4-heteroaryl-1-piperazinethiocarboxamides which are not known in the art. Four 4-(2-pyrazinyl)-1-piperazinethiocarboxamides have been described (Chem. Abstr. 49:384d and 11666b); however, there is no known report of antiatherosclerotic activity for these compounds.

Atherosclerosis is a form of arteriosclerosis characterized by lipid accumulation in and thickening of the arterial walls of both medium- and large-sized arteries. Arterial walls are thereby weakened, and the elasticity and effective internal size of the artery is decreased. Atherosclerosis is the most common cause of coronary artery disease and is of great medical importance since the occlusion of medium- and large-sized arteries diminishes the supply of blood to vital organs such as the heart muscles and the brain. The sequelae to atherosclerosis include ischemic heart disease, heart failure, life-threatening arrythmias, senility, and stroke.

The fact that cholesterol is a major component of atherosclerotic lesions or plaques has been known for more than 100 years. Various researchers have studied the role of cholesterol in lesion formation and development and also, more importantly, whether lesion formation can be prevented or lesion development arrested or reversed. Atheromatous lesions have now been shown [Adams, et al., Atherosclerosis, 13, 429 (1974)] to contain a greater quantity of esterified as opposed to unesterified cholesterol than the surrounding undiseased arterial wall. The intracellular esterification of cholesterol with fatty acids is catalyzed by the enzyme "Fatty acyl CoA:cholesterol acyl transferase" or ACAT, and the accumulation and storage of cholesterol esters in the arterial wall is associated with increased activity of this enzyme [Hashimoto and Dayton, Atherosclerosis, 28, 447 (1977)]. In addition, cholesterol esters are removed from the cells at a slower rate than unesterified cholesterol [Bondjers and Bjorkerud, Atherosclerosis, 15, 273 (1972) and 22, 379 (1975)]. Thus, inhibition of the ACAT enzyme would diminish the rate of cholesterol esterification, decrease the accumulation and storage of cholesterol esters in the arterial wall, and prevent or inhibit the formation and development of atheromatous lesions. The compounds of the present invention are very potent inhibitors of the ACAT enzyme. Thus, these compounds are useful for controlling and reducing the cholesterol ester content of mammalian arterial walls and decreasing the accumulation and storage of cholesterol in the arterial walls of mammals. Further, the compounds of this invention inhibit the formation or development of atherosclerotic lesions in mammals.

The evidence that hyperlipidemia is one of the factors involved in coronary heart disease is very impressive. A most important study carried out in Framingham, Mass. (Gordon and Verter, 1969) in over 5,000 persons for more than 12 years established a correlation between high concentrations of blood cholesterol and increased risk of heart attack. Although the causes of coronary artery disease are multiple, one of the most constant factors has been the elevated concentration of lipids in the blood plasma. A combined elevation of cholesterol and triglycerides has been shown (Carlson and Bottiger, 1972) to carry the highest risk of coronary heart disease. The majority of patients with ischemic heart disease or peripheral vascular disease were found to have hyperlipoproteinemia, involving very low-density and/or low-density lipoproteins (Lewis, et al., 1974).

We have now found that certain members of this class of compounds can safely and effectively lower serum lipids in warm-blooded animals. Such action on serum lipids is considered to be very useful in the treatment of atherosclerosis. For some time it has been considered desirable to lower serum-lipid levels and to correct lipoprotein imbalance in mammals as a preventive measure against atherosclerosis. The compounds of the present invention do not act by blocking late stages of cholesterol biosynthesis and thus do not produce accumulation of

intermediates such as desmosterol, as equally undesirable as cholesterol itself. Compounds with the combination of therapeutically-favorable characteristics possessed by those of the present invention can be safely administered to warm-blooded mammals for the treatment of hyperlipidemic and atherosclerotic states found in patients with or prone to heart attacks, to peripheral or cerebral vascular disease, and to stroke.

The compounds of this invention exhibit antiatherosclerotic activity, and the invention should not be construed as limited to any particular mechanism of antiatherosclerotic action.

This invention relates to new organic compounds, their preparation, pharmaceutical compositions containing them, and their use in the treatment of atherosclerosis. More particularly, it is concerned with novel compounds which may be represented by the formula:

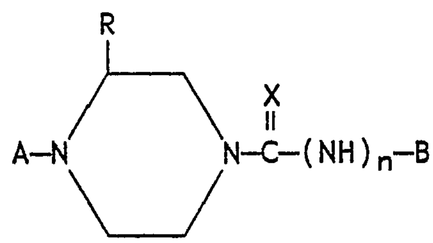

I

wherein X is oxygen or sulfur; and when X is oxygen: A is selected from the group consisting of 2-pyrazinyl, 2-quinoxalinyl, 2-quinolyl, p-chlorophenyl, 3-pyridylmethyl, 2-thiazolyl, and 6-methoxy-2-pyrazinyl; B is selected from the group consisting of phenyl, mono- and polysubstituted phenyl [wherein the substituents may be fluoro, alkyl ($C_1$-$C_4$), or trifluoromethyl], benzyl, mono- and disubstituted benzyl [wherein the substituents may be chloro, nitro, or alkyl ($C_1$-$C_3$)], alkyl ($C_1$-$C_7$), cycloalkyl ($C_3$-$C_6$), pyridyl, 2-phenoxypropionyl, alkyl($C_1$-$C_4$)amino, polyfluoro($F_7$-$F_{14}$)-alkyl($C_3$-$C_7$), mono- and disubstituted phenylamino (wherein the substituents may be chloro, carboxylic acid, or ethyl

carboxylate), 6-p-chlorophenylhexyl, 5-(p-chlorobenzoyl)-butyl, 2-p-chlorophenoxy-2-isopropyl, and 1-methylbenzyl; R is selected from the group consisting of hydrogen and alkyl ($C_1$-$C_3$); and n is zero. When X is sulfur: A is selected from the group consisting of pyrazinyl, quinolyl, and methoxypyrazinyl; B is selected from the group consisting of cycloalkyl, benzyl, phenethyl, and phenyl substituted with at least one substituent selected from the group consisting of $C_1$-$C_4$ carboalkoxy, carboxy, and cyano; R is hydrogen; and n is one.

This invention is further concerned with methods for treating atherosclerosis in a mammal with 1-piperazinecarbonyl compounds which may be represented by the formula:

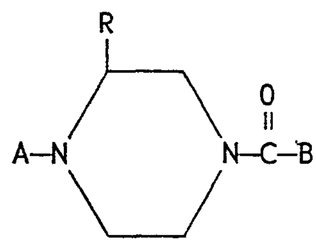

II

wherein R and B are as described above for the case in which X is oxygen and n is zero; and A, in addition to being as described above, may be unsubstituted phenyl.

This invention is further concerned with methods for treating atherosclerosis in a mammal with 1-piperazinethiocarboxamides which may be represented by the formula:

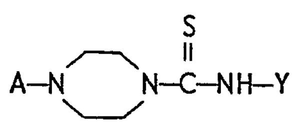

III

wherein A is selected from the group consisting of pyrazinyl, quinolyl, and methoxypyrazinyl; and Y is selected from the group consisting of $C_1$-$C_4$ alkyl, cycloalkyl,

benzyl, phenethyl, phenyl, and phenyl substituted with at least one substituent selected from the group consisting of $C_1$-$C_4$ alkyl, $C_1$-$C_4$ alkoxy, halo, $C_1$-$C_4$ carboalkoxy, carboxy, and cyano.

This invention also relates to a method of reducing the cholesterol content of the arterial walls of mammals which comprises administering to said mammal an effective amount of a compound of Formula II or III above.

This invention also relates to a method of treating hyperlipidemia in a mammal which comprises administering to said mammal an effective amount of a compound of Formula II or III above.

This invention also relates to a method of inhibiting atherosclerotic lesion development in a mammal which comprises administering to said mammal an effective amount of a compound of Formula II or III above.

This invention still further relates to a pharmaceutical composition which comprises an effective antiatherosclerotic amount of a compound of Formula II or II above in association with a pharmaceutically-acceptable carrier.

Finally, this invention relates to chemical processes for preparing the novel compounds of Formula I above. These processes are disclosed more completely in the detailed description of the invention below.

Preferred embodiments of the invention involve compounds of Formula I in which X is oxygen, n is zero, and A is a 2-pyrazinyl, 2-quinolyl, or 4-chlorophenyl group. More preferred embodiments of this type involve compounds of Formula I in which A is a 2-pyrazinyl, 2-quinolyl, or 4-chlorophenyl group and R is hydrogen or methyl. The most preferred embodiments of this type involve compounds of Formula I in which A is a 2-pyrazinyl, 2-quinolyl, or 4-chlorophenyl group; R is hydrogen or methyl, and B is mono- or disubstituted benzyl. Specific preferred embodiments of the invention involve the compounds: 1-(p-chlorophenyl)-4-phenylacetylpiperazine, 1-(p-chlorophenyl)-2-methyl-4-phenylacetylpiperazine, 1-(2-

quinolyl)-4-phenylacetylpiperazine, 1-[6-(p-chlorophenyl)-hexanoyl]-4-(2-pyrazinyl)piperazine, 1-(pentadecafluoro-octanoyl)-4-(2-pyrazinyl)piperazine, 1-(o-methylphenyl-acetyl)-4-(2-pyrazinyl)piperazine, 1-(o-methylphenylacet-yl)-4-(2-pyrazinyl)piperazine, 1-(2-pyrazinyl)-4-(p-tolyl-acetyl)piperazine, 1-(3,4-dichlorophenylacetyl)-4-(2-pyrazinyl)piperazine, 1-(2-pyrazinyl)-4-(α,α,α-trifluro-o-toluoyl)piperazine, and 1-(p-nitrophenylacetyl)-4-(2-pyrazinyl)piperazine.

A second class of preferred embodiments of the invention involve compounds of Formula I in which A is a 2-pyrazinyl group. More preferred embodiments of this type involve compounds of Formula I in which A is a 2-pyrazinyl group and B is a substituted phenyl group. The most preferred embodiments of this type involve compounds of Formula I in which A is a 2-pyrazinyl group and B is a phenyl group substituted with one cyano group. Specific preferred embodiments of the invention invovle the compound 4'-cyano-4-(2-pyrazinyl)-1-piperazinethiocarbox-anilide.

The compounds of Formula I wherein X is oxygen may be prepared by one of the following procedures:

A) An appropriately substituted phenylacetyl derivative and thionyl chloride in methylene chloride is refluxed for 1-2 hours, then the solvent is evaporated. The residue is dissolved in benzene and added dropwise to a solution of 1-(2-pyrazinyl)piperazine and triethylamine in benzene. This mixture is refluxed for 1-2 hours, filtered while hot, extracted with dilute alkali and evaporated giving the products of Examples 1-11, 35, and 36.

B) A 2-(1-piperazinyl)aryl derivative and triethylamine in benzene are added dropwise to a solution of phenylacetylchloride in benzene and refluxed for 1-2 hours. Evaporation and recrystallization from benzene gives the products of Examples 12-26 and 37.

C) An appropriately substituted phenylisocyanate in a solution in ether is added dropwise to a solution of 1-(2-pyrazinyl)piperazine in ether and stirred for 1-2 hours giving the products of Examples 27-32.

The compounds of Formula I wherein X is sulfur may be prepared by reactions of piperazines of Formula IV in which A is as defined above with isothiocyanates of Formula V in which B is as defined above.

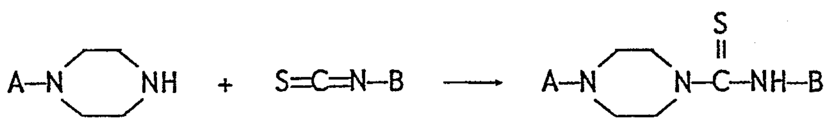

IV V

These reactions may be conducted in organic solvents such as ether, tetrahydrofuran, and methlene chloride at temperatures of from room temperature or below up to the boiling point of the solvent used and for reaction times of up to 24 hours or more. The resulting 1-piperazinethiocarboxamides are then isolated either by filtration or evaporation and purified by distillation under reduced pressure or by recrystallization from organic solvents such as ethanol, acetone, and benzene. An example of this process is the reaction of 1-(2-pyrazinyl)piperazine with 4-(cyanophenyl)isothiocyanate to yield 4'-cyano-4-(2-pyrazinyl)-1-piperazinethiocarboxanilide.

The piperazines of the present invention are obtained as crystalline solids or distillable liquids. The are characterized by distinct melting or boiling points and unique spectra. They are appreciably soluble in organic solvents but generally less soluble in water. The preparation and properties of the compounds of this invention will be described in greater detail in conjunction with the examples shown below.

The compounds of this invention were assayed for three types of biological activity related to their potential use as antiatherosclerotic agents.

The compounds of the present invention were tested for their ability to inhibit the enzymatic esterification of cholesterol according to the procedure of Hashimoto, et al., Life Sci., 12 (Part II), 1-12 (1973).

Rat adrenals were homogenized in 0.2M monobasic potassium phosphate buffer, pH 7.4, and centrifuged at 1,000 times gravity for 15 minutes at 5°C. The supernatant, containing the microsomal fraction, served as the source of the cholesterol-esterifying enzyme, fatty acyl CoA:cholesterol acyl transferase (ACAT). A mixture comprising 50 parts of adrenal supernatant, 10 parts of bovine serum albumin (50 mg./ml.), 3 parts of test compound (final concentration 5.2 µg./ml.), and 500 parts of buffer were preincubated at 37°C. for 30 minutes. A control mixture, omitting the test compound, was prepared and treated in the same manner. The lipids from the incubation mixture were extracted into an organic solvent and separated by thin-layer chromatography. The cholesterol ester fraction was counted in a scintillation counter. A statistically significant inhibition of the ACAT enzyme is the criterion for activity.

The results of this test on representative compounds of this invention appear in Table I.

TABLE I

| COMPOUND | % INHIBITION |
|---|---|
| 1-(2-phenoxypropionyl)-4-(2-pyrazinyl)piperazine | 30.5 |
| 1-phenylacetyl-4-(2-quinoxalinyl)piperazine | 40.1 |
| 1-(p-tert.-butylbenzoyl)-4-(2-pyrazinyl)-piperazine | 39.7 |
| 1-octanoyl-4-(2-pyrazinyl)piperazine | 33.6 |
| 1-(2,4-dichlorophenylacetyl)-4-(2-pyrazinyl)-piperazine | 32.2 |
| 1-(p-chlorophenyl)-4-phenylacetylpiperazine | 73.3 |
| 1-(3,4-dichlorophenylacetyl)-4-(2-pyrazinyl)-piperazine | 38.3 |
| 1-phenyl-4-phenylacetylpiperazine | 34.6 |
| 1-(p-nitrophenylacetyl)-4-(2-pyrazinyl)piperazine | 36.4 |
| 1-(p-chlorophenyl)-2-methyl-4-phenylacetyl-piperazine | 88.1 |
| 1-pentafluorobenzoyl-4-(2-pyrazinyl)piperazine | 31.2 |
| N-tert.-butyl-4-(2-pyrazinyl)-1-piperazine-carboxamide | 35.4 |
| ethyl-p-[4-(2-pyrazinyl)-1-piperazinecarboxamido]benzoate | 42.2 |
| 1-(2-thiazolyl)-4-o-tolylacetylpiperazine | 32.6 |
| 1-phenylacetyl-4-(2-quinolyl)piperazine | 89.7 |
| 1-[5-(p-chlorobenzoyl)valeryl]-4-(2-pyrazinyl)-piperazine | 47.4 |
| 1-[2-(p-chlorophenoxy)-2-methylpropionyl]-4-(2-pyrazinyl)piperazine | 54.1 |
| p-[4-(2-pyrazinyl)-1-piperazinecarboxamido] benzoic acid | 30.6 |
| 1-(pentadecafluorooctanoyl)-4-(2-pyrazinyl) piperazine | 39.0 |

0094498

TABLE I (continued)

| COMPOUND | % INHIBITION |
|---|---|
| 1-(6-methoxy-2-pyrazinyl)-4-phenylacetylpiperazine | 38.1 |
| 1-[6-(p-chlorophenyl)hexanoyl]-4-(2-pyrazinyl)piperazine | 68.9 |
| N-Phenethyl-4-(2-pyrazinyl)-1-piperazinethiocarboxamide | 33.0 |
| 4'-Isopropyl-4-(2-pyrazinyl)-1-piperazinethiocarboxanilide | 56.0 |
| N-Cyclohexyl-4-(2-pyrazinyl)-1-piperazinethiocarboxamide | 31.0 |
| 4'-Chloro-4-(2-pyrazinyl)-1-piperazinethiocarboxanilide | 31.0 |
| N-Ethyl-4-(2-quinolyl)-1-piperazinethiocarboxamide | 48.0 |
| N-Ethyl-4-(6-methoxy-2-pyrazinyl)-1-piperazinecarboxamide | 40.0 |

The compounds were tested for their ability to lower lipid levels in mammals. The compounds were administered orally admixed with diet to groups of four male rats COBS, CD, SD strain from Charles River Breeding Laboratories, Inc., Wilmington, Massachusetts. A control group of eight rats was maintained on the diet alone; test groups were maintained on the diet plus 0.1% of test compound by weight. After 5 days treatment, serum cholesterol and triglyceride concentrations were determined by direct enzymatic procedures using a Centrifichem® System 400 autoanalyzer (Union Carbide Co.). Cholesterol concentrations were determined by the combined cholesterol esterase-cholesterol oxidase procedure of Roeschlau, et al., Zeit. Klin. Chem. Klin. Biochem., 12, 226 (1974). Triglycerides were determined by the combined method of lipase catalyzed hydrolysis of triglycerides to glycerol and free fatty acids [Bucolo, G. and David, H., Clin. Chem., 19, 476 (1973) and Wahlefeld, A. W., in

"Methods of Enzymatic Analysis," Vol. 4, Bergmeyer, H. D., Editor, Academic Press, New York, NY (1974), pp. 1831-1835] and the enzymatic oxidation of the glycerol which leads to the production of colored formazan [Stavropoulos, W. S. and Crouch, R. D., Clin. Chem., 20, No. 7, 857 (1974)]. Changes in serum lipids are expressed as percent lowering from the values in control animals which did not receive drug treatment. The compounds which produced statistically significant lowering of either sterol or triglycerides are considered to be active. The results of this test on representative animals appear in Table II.

TABLE II

| | % LOWERING OF SERUM | |
|---|---|---|
| COMPOUND | STEROL | TRIGLYCERIDES |
| 1-(m-chlorophenylacetyl)-4-(2-pyrazinyl)piperazine | | 46 |
| 1-(pentadecafluorooctanoyl)-4-(2-pyrazinyl)piperazine | 47 | 74 |
| 1-(2-phenoxypropionyl)-4-(2-pyrazinyl)piperazine | | 62 |
| 1-(2-pyrazinyl)-4-(3-pyridylcarbonyl)piperazine | | 30 |
| 1-(2,4-dichlorophenylacetyl)-4-(2-pyrazinyl)piperazine | | 44 |
| 1-(2-pyrazinyl)-4-(p-tolylacetyl) piperazine | | 49 |
| 1-(2-pyrazinyl)-4-(m-tolylacetyl) piperazine | | 40 |
| 1-(2-methylphenylacetyl)-4-(2-pyrazinyl)piperazine | | 55 |
| 1-(p-chlorophenyl)-2-methyl-4-phenylacetylpiperazine | | 61 |
| 1-(p-chlorophenylacetyl)-4-(2-pyrazinyl)piperazine | | 51 |

TABLE II (continued)

| COMPOUND | % LOWERING OF SERUM STEROL | % LOWERING OF SERUM TRIGLYCERIDES |
|---|---|---|
| 1-phenylacetyl-4-(3-pyridylmethyl) piperazine | | 34 |
| N-ethyl-4-(2-pyrazinyl)-1-piperazinecarboxamide | | 43 |
| N-methyl-4-(2-pyrazinyl)-1-piperazinecarboxamide | | 38 |
| 4'-chloro-4-(2-pyrazinyl)-1-piperazinecarboxanilide | | 33 |
| 1-benzoyl-4-(2-pyrazinyl)piperazine | | 30 |
| 1-heptafluorobutyroyl-4-(2-pyrazinyl)piperazine | | 51 |
| 1-cyclopropylcarbonyl-4-(2-pyrazinyl)piperazine | | 37 |
| 1-phenylacetyl-4-(2-quinolyl) piperazine | 69 | 53 |
| 1-[5-(p-chlorobenzoyl)valeryl]-4-(2-pyrazinyl)piperazine | | 45 |
| 1-[2-(p-chlorophenoxy)-2-methylpropionyl]-4-(2-pyrazinyl)piperazine | | 42 |
| 1-(6-methoxy-2-pyrazinyl)-4-phenylacetylpiperazine | | 56 |
| 1-[6-(p-chlorophenyl)hexanoyl]-4-(2-pyrazinyl)piperazine | | 30 |
| N-Methyl-4-(2-pyrazinyl)-1-piperazinethiocarboxamide | | 31 |
| 4'-Cyano-4-(2-pyrazinyl)-1-piperazinethiocarboxanilide | | 55 |
| N-Phenethyl-4-(2-pyrazinyl)-1-piperazinethiocarboxamide | 16 | |
| N-Phenyl-4-(2-pyrazinyl)-1-piperazinethiocarboxamide | | 52 |

TABLE II (continued)

| COMPOUND | % LOWERING OF SERUM STEROL | % LOWERING OF SERUM TRIGLYCERIDES |
|---|---|---|
| 2',6'-Dichloro-4-(2-pyrazinyl)-1-piperazinethiocarboxanilide | | 37 |
| 2'-Chloro-4-(2-pyrazinyl)-1-piperazinethiocarboxanilide | | 49 |
| 2'-Fluoro-4-(2-pyrazinyl)-1-piperazinecarboxanilide | | 21 |
| N-Cyclohexyl-4-(2-pyrazinyl)-1-piperazinethiocarboxamide | | 43 |
| 4'-Chloro-4-(2-pyrazinyl)-1-piperazinethiocarboxanilide | | 49 |
| 4'-Fluoro-4-(2-pyrazinyl)-1-piperazinethiocarboxanilide | | 37 |
| N-Ethyl-4-(6-methoxy-2-pyrazinyl)-1-piperazinecarboxamide | | 38 |

The compounds were further tested for their ability to decrease aortic sterol content without effecting serum sterol.

Day-old cockerels were placed on diets consisting of pullet starter mash supplemented with either 20 g. cholesterol (control group) or 20 g. cholesterol and 525 mg. test compound (drug-treated group) per kg. of diet.

For the control diet, 20 g. of cholesterol was dissolved in 200 ml. of chloroform, mixed into 1 kg. of pullet starter mash, and then the chloroform was removed by evaporation.

For the drug-treatment group, 525 mg. of the test compound was dissolved in 100 ml. of chloroform, mixed into a diet prepared as described above, and then the chloroform was evaporated.

The cockerels were housed three per cage and given water and their respective diet *ad libitum* for 14 days. Blood was collected by cardiac puncture, and the serum was saponified [Trinder, P., Analyst, 77, 321 (1952)] and extracted and the cholesterol content determined [Zlatkis, A., et al., J. Lab. Clin. Med., 41, 486 (1953)]. The aortae were removed, cleaned of adventitial tissue, and the sterol content determined according to the above procedures. Compounds which statistically decrease the aortic sterol content without having an effect on serum sterol are considered to be active. The results of this test on representative compounds of this invention appear in Table III.

TABLE III

| COMPOUND | RESULT |
|---|---|
| 1-(*o*-methylphenylacetyl)-4-(2-pyrazinyl)piperazine | Active |
| 1-(2-pyrazinyl)-4-(*p*-tolylacetyl)piperazine | Active |
| 1-(3,4-dichlorophenylacetyl)-4-(2-pyrazinyl) piperazine | Active |
| 1-(2-pyrazinyl)-4-(α,α,α-trifluoro-*o*-toluoyl) piperazine | Active |
| 1-phenyl-4-phenylacetyl piperazine | Active |
| 1-(*p*-nitrophenylacetyl)-4-(2-pyrazinyl)piperazine | Active |
| 2',6'-dichloro-4-(2-pyrazinyl)-1-piperazinecarboxanilide | Active |
| N-methyl-4-(2-pyrazinyl)-1-piperazinethiocarboxamide | Active |
| 4'-cyano-4-(2-pyrazinyl)-1-piperazinethiocarboxanilide | Active |
| N-phenyl-4-(2-pyrazinyl)-1-piperazinethiocarboxamide | Active |
| N-ethyl-4-(2-pyrazinyl)-1-piperazinethiocarboxamide | Active |

When the compounds are employed for the above utility, they may be combined with one or more pharmaceutically-acceptable carriers, e.g., solvents, diluents, and the like, and may be administered orally in such forms as tablets, capsules, dispersible powders, granules, suspensions containing, for example, from about 0.5% to 5% of suspending agent, syrups containing, for example, from about 10% to 50% of sugar, and elixirs containing, for example, from about 20% to 50% ethanol, and the like, or parenterally in the form of sterile injectable solutions or suspensions containing from about 0.5% to 5% suspending agent in an isotonic medium. These pharmaceutical preparations may contain, for example, from about 0.5% up to about 90% of the active ingredient in combination with the carrier, more usually between 5% and 60% by weight.

The antiatherosclerotic effective dosage of active ingredient employed for the reduction of cholesterol ester content in the arterial walls of a mammal may vary depending on the particular compound employed, the mode of administration, and the severity of the condition being treated. In general, however, satisfactory results are obtained when the compounds of the invention are administered at a daily dosage of from about 2 mg. to about 500 mg. per kg. of animal body weight, preferably given in divided doses two to four times a day, or in sustained release form. For most large mammals, the total daily dosage is from about 100 mg. to about 5,000 mg., preferably from about 100 mg. to 2,000 mg. Dosage forms suitable for internal use comprise from about 25 to 500 mg. of the active compound in intimate admixture with a solid or liquid pharmaceutically-acceptable carrier. This dosage regimen may be adjusted to provide the optimal thereapeutic response. For example, several divided doses may be administered daily, or the dose may be proportionally reduced as indicated by the exigencies of the therapeutic situation. A decided practical advantage is that these active compounds may be administered orally as well as by intravenous, intramuscular, or subcutaneous routes if necessary. Solid carriers include starch, lactose, dicalcium phosphate, microcrystalline cellulose, sucrose, and kaolin, while liquid carriers include sterile water, polyethylene glycols, nonionic surfactants, and edible oils such as corn, peanut, and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, e.g., vitamin E, ascorbic acid, BHT, and BHA.

The preferred pharmaceutical compositions from the stand-point of ease of preparation and administration are solid compositions, particularly tablets and hard-filled or liquid-filled capsules. Oral administration of the compounds is preferred.

These active compounds may also be administered parenterally or intraperitoneally. Solutions or suspensions of these active compounds as a free base or pharmacologically-acceptable salt can be prepared in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils.

The invention will be more fully described in conjunction with the following specific examples which are not to be construed as limiting the scope of the invention.

Example 1

1-(2,4-Dichlorophenylacetyl)-4-(2-pyrazinyl)piperazine

A solution of 6.15 g. of 2,4-dichlorophenylacetic acid and 22.0 ml. of thionyl chloride in 20 ml. of methylenechloride was refluxed for 1 hour. The solvent was evaporated, and a solution of the residue in 30 ml. of benzene was added dropwise with stirring to a solution of 4.92 g. of 1-(2-pyrazinyl)piperazine and 3.80 g. of triethylamine in 60 ml. of benzene. The mixture was refluxed for 1 hour, then filtered while hot. The filtrate was extracted with dilute sodium hydroxide, washed with water, dried, evaporated, and recrystallized from ethanol, giving 7.43 g. of the desired product, m.p. 124-125°C.

The products listed as Examples 2-11 in Table IV were prepared by the procedure of Example 1, employing appropriate starting materials.

TABLE IV

| Example | Name | Crystallization Solvent | M.P. °C. |
| --- | --- | --- | --- |
| 2 | 1-(*m*-Chlorophenylacetyl)-4-(2-pyrazinyl)-piperazine | methanol | 84-86 |
| 3 | 1-(*o*-Methylphenylacetyl)-4-(2-pyrazinyl)-piperazine | ethyl acetate: hexane | 92-94 |
| 4 | 1-(2-Pyrazinyl)-4-(*p*-tolylacetyl)piperazine | ethanol:water | 82-83 |
| 5 | 1-(3,4-Dichlorophenylacetyl)-4-(2-pyrazinyl)piperazine | acetonitrile | 125-126 |
| 6 | 1-(2-Pyrazinyl)-4-(*m*-tolylacetyl)piperazine | methylene chloride:hexane | 88-89 |
| 7 | 1-(*p*-Nitrophenylacetyl)-4-(2-pyrazinyl)-piperazine | ethyl acetate | 124-126 |
| 8 | 1-(2-Methylphenylacetyl)-4-(2-pyrazinyl)-piperazine | ethyl acetate: hexane | 95-96 |
| 9 | 1-(*p*-Chlorophenylacetyl)-4-(2-pyrazinyl)-piperazine | ethyl acetate | 130-132 |

TABLE IV (continued)

| Example | Name | Crystallization Solvent | M.P.°C. |
|---|---|---|---|
| 10 | 1-Heptafluorobutyroyl-4-(2-pyrazinyl)-piperazine | | oil |
| 11 | 1-[2-(p-Chlorophenoxy)-2-methylpropionyl]-4-(2-pyrazinyl)piperazine | ethyl acetate | 99-100 |

1 Example 12

1-Phenylacetyl-4-(2-quinoxalinyl)piperazine

To a solution of 6.42 g. of 2-(1-piperazinyl)-quinoxaline and 3.65 g. of triethylamine in 60 ml. of benzene was added dropwise with stirring a solution of 4.64 g. of phenylacetylchloride in 30 ml. of benzene. The mixture was refluxed for 1 hour, then filtered while hot. Evaporation of the filtrate and recrystallization from benzene gave 5.2 g. of the desired product, m.p. 156-158°C.

The products listed as Examples 13-26 in Table V were prepared by the procedure of Example 12, employing appropriate starting materials.

TABLE V

| Example | Name | Crystallization Solvent | M.P.°C. |
|---|---|---|---|
| 13 | 1-(2-Phenoxypropionyl)-4-(2-pyrazinyl)-piperazine | methanol | 114-115 |
| 14 | 1-(*p*-*tert*.-Butylbenzoyl)-4-(2-pyrazinyl)-piperazine | ethanol:water | 140-141 |
| 15 | 1-Octanoyl-4-(2-pyrazinyl)piperazine | ethanol:water | 72-73 |
| 16 | 1-(2-Pyrazinyl)-4-(3-pyridylcarbonyl)-piperazine | methylene chloride:hexane | 87-89 |
| 17 | 1-(2-Pyrazinyl)-4-(α,α,α-trifluoro-*o*-toluoyl)piperazine | ethanol | 123-124 |
| 18 | 1-Phenyl-4-phenylacetylpiperazine | ethyl acetate: hexane | 93-94 |
| 19 | 1-(*p*-Chlorophenyl)-2-methyl-4-phenyl-acetylpiperazine | ethanol | 122-123 |
| 20 | 1-Phenylacetyl-4-(3-pyridylmethyl)-piperazine | | oil |
| 21 | 1-Benzoyl-4-(2-pyrazinyl)piperazine | ethanol:water | 109-111 |
| 22 | 1-Cyclopropylcarbonyl-4-(2-pyrazinyl)-piperazine | ethyl acetate: hexane | 94-95 |

TABLE V (continued)

| Example | Name | Crystallization Solvent | M.P.°C. |
|---|---|---|---|
| 23 | 1-Pentafluorobenzoyl-4-(2-pyrazinyl)-piperazine | ethanol:water | 120-121 |
| 24 | 1-Phenylacetyl-4-(2-quinolyl)piperazine | acetonitrile | 135-138 |
| 25 | 1-(Pentadecafluorooctanoyl)-4-(2-pyrazinyl)piperazine | 80% ethanol | 82-84 |
| 26 | 1-(6-Methoxy-2-pyrazinyl)-4-phenylacetyl-piperazine | ethyl acetate | 133-135 |

Example 27

2',6'-Dichloro-4-(2-pyrazinyl)-1-piperazinecarboxanilide

A solution of 1.88 g. of 2,6-dichlorophenylisocyanate in 25 ml. of anhydrous ether was added dropwise to a stirred solution of 1.64 g. of 1-(2-pyrazinyl)piperazine in 250 ml. of anhydrous ether. The mixture was stirred for 30 minutes, and the solid was collected and recrystallized from ethanol, giving 3.12 g. of the desired product, m.p. 177-179°C.

The products listed as Examples 28-32 in Table VI were prepared by the procedure of Example 27, employing appropriate starting materials.

TABLE VI

| Example | Name | Crystallization Solvent | M.P. °C. |
| --- | --- | --- | --- |
| 28 | N-Ethyl-4-(2-pyrazinyl)-1-piperazine-carboxamide | ethanol | 169.5-171.5 |
| 29 | N-Methyl-4-(2-pyrazinyl)-1-piperazine-carboxamide | ethanol | 120-122 |
| 30 | 4'-Chloro-4-(2-pyrazinyl)-1-piperazine-carboxanilide | ethanol | 155-157 |
| 31 | N-*tert*.-Butyl-4-(2-pyrazinyl)-1-piperazinecarboxamide | ethanol | 201-203 |
| 32 | Ethyl-*p*-[4-(2-pyrazinyl)-1-piperazine-carboxamido]benzoate | benzene | 163-165 |

Example 33

1-(p-Chlorophenyl)-4-phenylacetylpiperazine

To a cold solution of 8.09 g. of p-chlorophenylpiperazine dihydrochloride and 40 ml. of 10% sodium hydroxide in 60 ml. of water was added dropwise with stirring 4.64 g. of phenylacetyl chloride. The mixture was stirred for 1 hour, then neutralized with 2N hydrochloric acid. The solid was collected and recrystallized from ethanol-water, giving 4.2 g. of the desired product, m.p. 93-94°C.

Example 34

p-[4-(2-Pyrazinyl)-1-piperazinecarboxamido]benzoic acid

A solution of 6.69 g. of ethyl p-[4-(2-pyrazinyl)-1-piperazinecarboxamido]benzoate and 1.32 g. of potassium hydroxide in 90 ml. of 80% ethanol was refluxed for 18 hours. The mixture was cooled and diluted with 90 ml. of water, then acidified with concentrated hydrochloric acid. The solid was collected and recrystallized from isopropanol, giving 3.5 g. of the desired product, m.p. 243-245°C.

Example 35

1-(2-Thiazolyl)-4-o-tolylacetylpiperazine

A mixture of 20.5 g. of 2-bromothiazole, 15.0 g. of anhydrous piperazine, 15.0 g. of sodium carbonate, and 30 ml. of amyl alcohol was heated at reflux for 4 hours, then allowed to stand at ambient temperature for 48 hours, and filtered. The filtrate was extracted with four 10 ml. portions of 3N hydrochloric acid. The extracts were combined and the aqueous layer made basic with 10N sodium hydroxide to pH 12-13. The mixture was filtered and the filtrate extracted with three 50 ml. portions of chloroform. These extracts were combined, dried, and concentrated in vacuo to a yellow oil. This oil was distilled on a Kugelrohr apparatus at 90°C./20 mm., giving 16.79 g. of 1-(2-thiazolyl)piperazine as a colorless oil.

The 1-(2-thiazolyl)piperazine was converted to the desired product in 57% yield, by the procedure of Example 1 using the appropriate starting material, upon recrystallization from ethanol, m.p. 110-111°C.

Example 36

1-[5-(p-Chlorobenzoyl)valeryl]-4-(2-pyrazinyl)piperazine

5-p-chlorobenzoylvaleric acid [L. F. Fieser, et al., J. Am. Chem. Soc., 70, 3197 (1948)] was reacted as described in Example 1, giving 74% yield of the desired product when recrystallized from ethanol, m.p. 138-140°C.

Example 37

1-[6-(p-Chlorophenyl)hexanoyl]-4-(2-pyrazinyl)piperazine

To a solution of 101 g. of ethyl adipate in 500 ml. of benzene was slowly added 120 ml. of thionyl chloride. The mixture was refluxed 4.5 hours, cooled, and evaporated. The residue was evaporated three times from 500 ml. of benzene, giving 113 g. of yellow liquid.

A mixture of 155 g. of aluminum chloride, 200 ml. of dichloromethane, and 77 ml. of p-chlorophenol was cooled to 3°C. in an ice-water bath. The above 113 g. of yellow liquid was added slowly with stirring over a period of 3 hours while the temperature was maintained at less than 5°C. The mixture was refrigerated overnight, then warmed to 50°C., stirred for 1 hour, and poured slowly into 1.5 liters of ice water containing 200 ml. of 37% hydrochloric acid. The resulting oil was collected and evaporated giving 159.1 g. of tan solid. To a solution of this solid in 400 ml. of ethanol was slowly added 80 g. of 85% potassium hydroxide. After standing 1/2 hour, 100 ml. of water was added, the solution was stirred and refluxed for 2 hours, then cooled, and evaporated. The residue was dissolved in one liter of water, adjusted to pH 1 with 37% hydrochloric acid, and the solid was collected and recrystallized twice from ethanol giving 69.5 g. of orange solid.

A 55 g. portion of zinc was combined with 5.4 g. of mercuric chloride, 90 ml. of water, and 3 ml. of concentrated hydrochloric acid. To this was sequentially added 35 ml. of water, 80 ml. of 37% hydrochloric acid, 45 ml. of toluene, and 35 g. of the above orange solid. The reaction was brought to reflux for 24 hours, with 25 ml. portions of 37% hydrochloric acid added at 6-hour intervals. The reaction was cooled,

diluted with water, and the organic layer separated and distilled on a Kugelrohr apparatus, giving (138-142°C./50 mm.) 26.3 g. of colorless liquid. This liquid was hydrogenated with palladium on carbon catalyst giving 25.2 g. of colorless liquid. A solution of 15 g. of this liquid and 14 ml. of sulfonyl chloride in 200 ml. of benzene was refluxed for 6 hours, cooled, evaporated, and extracted with three 200 ml. portions of benzene, giving 16.7 g. of 6-(p-chlorophenyl)hexanoyl chloride as an orange oil.

The 6-(p-chlorophenyl)hexanoyl chloride was reacted as described in Example 12 giving a 70% yield of the desired product when recrystallized from ethyl acetate, m.p. 88-89°C.

## Example 38

### N-Methyl-4-(2-pyrazinyl)-1-piperazinethiocarboxamide

To a solution of 6.56 g. of 1-(2-pyrazinyl)piperazine (U.S. Patent 2,606,906) in 40 ml. of anhydrous ether is added a solution of 2.92 g. of methyl isothiocyanate in 40 ml. of anhydrous ether dropwise over a period of 10 minutes. This suspension is stirred for 1 hour, and the solid is collected and washed. This solid is dissolved in a mixture of 200 ml. of benzene and 50 ml. of ethanol with heat and then cooled to room temperature over 48 hours. The resulting solid is dissolved in 400 ml. of ethanol:ethyl acetate (1:3), concentrated to 200 ml. and chilled. The resulting solid is collected, giving 4.34 g. of the desired product as white crystals, m.p. 197-200°C.

## Example 39

### 4'-Cyano-4-(2-pyrazinyl)-1-piperazinethiocarboxanilide

To a solution of 4.92 g. of 1-(2-pyrazinyl)piperazine in 45 ml. of anhydrous ether is added a slurry of 4.8 g. of p-cyanophenyl isothiocyanate in 45 ml. of anhydrous ether dropwise over 10 minutes. The mixture is stirred for 30 minutes and the resulting solid collected. This solid is dissolved in a mixture of 200 ml. of benzene and 200 ml. of ethanol and then cooled. The resulting solid is collected, giving 6.95 g. of the desired product, m.p. 208-210°C.

Example 40

N-Phenethyl-4-(2-pyrazinyl)-1-piperazinethiocarboxamide

To a solution of 4.92 g. of 1-(2-pyrazinyl)piperazine in 30 ml. of anhydrous ether is added dropwise a solution of 4.89 g. of phenethyl isothiocyanate in 30 ml. of anhydrous ether over 10 minutes. An additional 30 ml. of anhydrous ether is required to maintain stirring over 30 minutes. The resulting solid is collected, dissolved in 100 ml. of benzene and 25 ml. of ethanol, and cooled. The resulting solid is collected, giving 5.68 g. of the desired product as a white solid, m.p. 162-163°C.

Example 41

2',6'-Dichloro-4-(2-pyrazinyl)-1-piperazinethiocarboxanilide

To a solution of 4.92 g. of 1-(2-pyrazinyl)piperazine in 50 ml. of anhydrous ether is added dropwise a solution of 6.12 g. of 2,6-dichlorophenyl isothiocyanate in 50 ml. of anhydrous ether over 10 minutes. The mixture is stirred for 15 minutes and the resulting solid collected. This solid is dissolved with heat in a mixture of 200 ml. of acetone and 150 ml. of methanol and then cooled. The resulting solid is collected, giving 5.29 g. of the desired product as white crystals, m.p. 225-227°C.

Example 42

2'-Chloro-4-(2-pyrazinyl)-1-piperazinethiocarboxanilide

To a solution of 4.92 g. of 1-(2-pyrazinyl)piperazine in 50 ml. of anhydrous ether is added dropwise a solution of 5.08 g. of 2-chlorophenyl isothiocyanate in 50 ml. of anhydrous ether over 5 minutes. The mixture is stirred for 30 minutes, and the resulting solid is collected. This solid is recrystallized from 430 ml. of ethanol, giving 7.41 g. of the desired product, m.p. 167-169°C.

Example 43

4'-Isopropyl-4-(2-pyrazinyl)-1-piperazinethiocarboxanilide

To a solution of 4.92 g. of 1-(2-pyrazinyl)piperazine in 50 ml. of anhydrous ether is added dropwise a solution of 5.31 g. of 4-isopropylphenyl isothiocyanate in 50 ml. of

anhydrous ether over 5 minutes. The mixture is stirred for 30 minutes, and the resulting solid is collected and recrystallized from 150 ml. of ethanol, giving 9.31 g. of the desired product as white crystals, m.p. 167-171°C.

Example 44

N-Cyclohexyl-4-(2-pyrazinyl)-1-piperazinethiocarboxamide

To a solution of 4.92 g. of 1-(2-pyrazinyl)piperazine in 50 ml. of anhydrous ether is added dropwise a solution of 4.2 g. of cyclohexyl isothiocyanate in 50 ml. of anhydrous ether over 5 minutes. The mixture is stirred for 35 minutes, and the resulting solid is collected and recrystallized from 50 ml. of ethanol, giving 7.58 g. of the desired product, m.p. 176-178°C.

Example 45

4'-Chloro-4-(2-pyrazinyl)-1-piperazinethiocarboxanilide

To a solution of 4.92 g. of 1-(2-pyrazinyl)piperazine in 50 ml. of anhydrous ether is added dropwise a solution of 5.08 g. of 4-chlorophenyl isothiocyanate in 50 ml. of anhydrous ether over a period of 5 minutes. The mixture is stirred for 30 minutes, and the resulting solid is collected and recrystallized from 550 ml. of ethanol, giving 7.92 g. of the desired product as white crystals, m.p. 208-210°C.

Example 46

4'-Fluoro-4-(2-pyrazinyl)-1-piperazinethiocarboxanilide

To a solution of 4.92 g. of 1-(2-pyrazinyl)piperazine in 50 ml. of anhydrous ether is added dropwise a solution of 4.59 g. of 4-fluorophenyl isothiocyanate in 50 ml. of anhydrous ether with stirring. Stirring is continued for 30 minutes, and the resulting solid is collected. This solid is dissolved in a mixture of 200 ml. of ethanol and 75 ml. of acetone with heat, then concentrated to 200 ml., and cooled, giving 7.43 g. of the desired product as a white solid, m.p. 195-197°C.

Example 47

N-Ethyl-4-(2-quinolyl)-1-piperazinethiocarboxamide

A mixture of 20.7 g. of 2-chloroquinoline, 15 g. of anhydrous piperazine, 15 g. of powdered sodium carbonate, and

30 ml. of anhydrous ethanol is stirred and refluxed for 8 hours in a flask equipped with a Dean-Stark trap condenser. The mixture is diluted with 100 ml. of chloroform and filtered. The filtrate is extracted twice with water, dried over magnesium sulfate, filtered, and the filtrate is concentrated *in vacuo* to a brown semi-liquid. A 200 ml. portion of chloroform is added, and the mixture is filtered. The filtrate is extracted with four 10 ml. portions of 3N hydrochloric acid. The extracts are combined, and the chloroform layer is saved. The aqueous layer is made basic with 10N sodium hydroxide to pH 13 and then extracted with three 50 ml. portions of chloroform. The chloroform extracts are combined, washed with two 5 ml. portions of water, dried and concentrated *in vacuo* to a solid. This solid is dissolved in 30 ml. of methanol and filtered. To the filtrate is added 60 ml. of water. The resulting solid is collected and dried, giving 16.69 g. of 1-(2-quinolinyl)piperazine.

To a slurry of 4.26 g. of 1-(2-quinolinyl)piperazine in 30 ml. of anhydrous ether is added dropwise a solution of 1.74 g. of ethyl isothiocyanate in 30 ml. of anhydrous ether. The mixture is stirred for 1 hour, and the resulting solid is collected and recrystallized from 150 ml. of ethanol, giving 4.43 g. of the desired product as a white crystalline solid, m.p. 193-195°C.

## Example 48

### N-Ethyl-4-(6-methoxy-2-pyrazinyl)-1-piperazinethiocarboxamide

A mixture of 29.8 g. of 2,6-dichloropyrazine, 17.2 g. of piperazine, and 200 ml. of acetone is refluxed on a steam bath for 2 hours, then cooled, and the resulting solid is collected, dried, and recrystallized from 80% ethanol, giving 26.0 g. of 1-[2-(6-chloropyrazinyl)]piperazine hydrochloride.

A mixture of 9.4 g. of 1-[2-(6-chloropyrazinyl)]-piperazine hydrochloride, 4.4 g. of sodium methoxide, and 120 ml. of methanol is refluxed for 19 hours, cooled, and diluted with 120 ml. of water. The mixture is extracted with four 25 ml. portions of chloroform which are combined, dried, and concentrated in vacuo to a residue. This residue is dissolved in 50 ml. of methanol, 2.2 g. of sodium methoxide are added, and the mixture is refluxed overnight, cooled, and 50 ml. of water are added. This mixture is extracted with four 20 ml. portions of chloroform, dried, and evaporated to a yellow oil. This oil is redissolved in 50 ml. of methanol, and a solution of 2.2 g. of sodium methoxide in 20 ml. of methanol is added. This mixture is refluxed for 40 hours, diluted with 50 ml. of water, and extracted with two 30 ml. portions of chloroform. The extracts are combined, dried, and concentrated to dryness, giving 6.78 g. of 1-(6-methoxypyrazinyl)-piperazine as a yellow oil.

To a solution of 1.94 g. of 1-(6-methoxypyrazinyl)piperazine in 25 ml. of anhydrous ether is added dropwise a solution of 0.87 g. of ethyl isothiocyanate in 25 ml. of anhydrous ether. The mixture is stirred 30 minutes and the solid collected and recrystallized from 80% ethanol, giving 2.27 g. of the desired product as white crystals, m.p. 120-122°C.

## Example 49

## 2'-Fluoro-4-(2-pyrazinyl)-1-piperazinethiocarboxanilide

To a solution of 4.92 g. of 1-(2-pyrazinyl)piperazine in 50 ml. of anhydrous ether is added a solution of 4.59 g. of 2-fluorophenyl isothiocyanate in 50 ml. of anhydrous ether over a period of 10 minutes. The mixture is stirred for 30 minutes, and the resulting solid is collected and then recrystallized from 200 ml. of ethanol, giving 7.04 g. of the desired product, m.p. 151-153°C.

## Example 50

N-Butyl-4-(2-pyrazinyl)-1-piperazinethiocarboxamide

To a solution of 4.92 g. of 1-(2-pyrazinyl)piperazine in 80 ml. of anhydrous ether is added a solution of 3.45 g. of *n*-butyl isothiocyanate in 50 ml. of anhydrous ether, dropwise during 5 minutes. The mixture is stirred for 30 minutes, and the resulting solid is collected and dissolved in 50 ml. of ethanol. Cooling and the addition of 2 ml. of water gives 4.96 g. of the desired product as white crystals, m.p. 97.5-98.5°C.

## Example 51

N-*tert*-Butyl-4-(2-pyrazinyl)-1-piperazinethiocarboxamide

To a solution of 4.92 g. of 1-(2-pyrazinyl)piperazine in 50 ml. of anhydrous ether is added a solution of 3.45 g. of *tert*-butyl isothiocyanate in 50 ml. of anhydrous ether. The mixture is stirred for 30 minutes, and the resulting solid is collected and recrystallized from 50 ml. of ethanol, giving 5.85 g. of the desired product as white crystals, m.p. 153-156°C.

864

We Claim:

1. A compound of the formula:

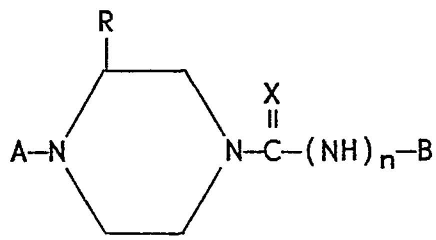

wherein X is oxygen or sulfur, and when X is oxygen: A is 2-pyrazinyl, 2-quinoxalinyl, 2-quinolyl, p-chlorophenyl, 3-pyridylmethyl, 2-thiazolyl, or 6-methoxy-2-pyrazinyl; B is phenyl, mono- or polysubstituted phenyl [wherein the substituents may be fluoro, alkyl ($C_1$-$C_4$), or trifluoromethyl], benzyl, mono- or disubstituted benzyl [wherein the substituents may be chloro, nitro, or alkyl ($C_1$-$C_3$)], alkyl ($C_1$-$C_7$), cycloalkyl ($C_3$-$C_6$), pyridyl, 2-phenoxypropionyl, alkyl($C_1$-$C_4$)amino, polyfluoro($F_7$-$F_{14}$)polyalkyl($C_3$-$C_7$), mono- or disubstituted phenylamino (wherein the substituents may be chloro, carboxy, or carboethoxy), 6-p-chlorophenylhexyl, 5-(p-chlorobenzoyl)butyl, 2-p-chlorophenoxy-2-isopropyl, or 1-methylbenzyl; R is hydrogen or alkyl ($C_1$-$C_3$); and n is zero; and when X is sulfur: A is pyrazinyl, quinolyl, or methoxypyrazinyl; B is cycloalkyl, benzyl, phenethyl, or phenyl substituted with at least one substituent [wherein the substituents may be carboalkoxy ($C_1$-$C_4$), carboxy, or cyano]; R is hydrogen; and n is one.

2. A compound of the formula:

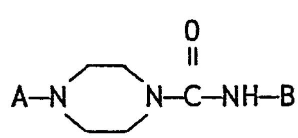

wherein A is 2-pyrazinyl or 2-thiazolyl, and B is phenyl or substituted phenyl [wherein the substituents may be alkyl ($C_1$-$C_4$), alkoxy ($C_1$-$C_4$), or halo].

3. The compound according to claim 1, 4'-chloro--4-(2-pyrazinyl)-1-piperazinecarboxanilide.

4. The compound as recited in claim 1, 4'-cyano-4-(2-pyrazinyl)-1-piperazinethiocarboxanilide.

5. A method of treating atherosclerosis in a mammal in need of such treatment which comprises administering to said mammal an effective antiatherosclerotic amount of a compound of the formula:

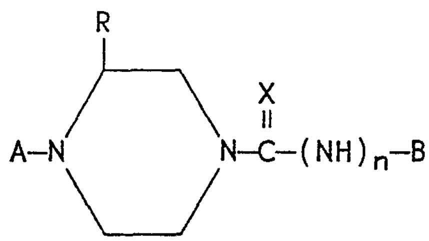

wherein X is oxygen or sulfur, and when X is oxygen: A is phenyl, 2-pyrazinyl, 2-quinoxalinyl, 2-quinolyl, p-chlorophenyl, 3-pyridylmethyl, 2-thiazolyl, or 6-methoxy-2-pyrazinyl; B is phenyl, mono- or polysubstituted phenyl [wherein the substituents may be fluoro, alkyl ($C_1$-$C_4$), or trifluoromethyl], benzyl, mono- or disubstituted benzyl [wherein the substituents may be chloro, nitro, or alkyl ($C_1$-$C_3$)], alkyl ($C_1$-$C_7$), cycloalkyl ($C_3$-$C_6$), pyridyl, 2-phenoxypropionyl, alkyl($C_1$-$C_4$)amino, polyfluoro($F_7$-$F_{14}$)polyalkyl($C_3$-$C_7$), mono- or disubstituted phenylamino (wherein the substituents may be chloro, carboxy, or carboethoxy), 6-p-chlorophenoxy-2-isopropyl, or 1-methylbenzyl; R is hydrogen or alkyl ($C_1$-$C_3$); and n is zero; and when X is sulfur: A is pyrazinyl, quinolyl, or methoxypyrazinyl; B is alkyl ($C_1$-$C_4$), cycloalkyl, benzyl, phenethyl, phenyl, or phenyl substituted with at least one substituent [wherein the substituents may be alkyl ($C_1$-$C_4$), alkoxy ($C_1$-$C_4$), halo, carboalkoxy ($C_1$-$C_4$), carboxy, or cyano]; R is hydrogen; and n is one.

6. A pharmaceutical composition in dosage unit form which comprises between 5% and 60% by weight of a compound of the formula:

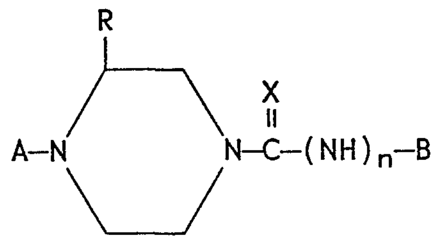

wherein X is oxygen or sulfur, and when X is oxygen: A is phenyl, 2-pyrazinyl, 2-quinoxalinyl, 2-quinolyl, p-chlorophenyl, 3-pyridylmethyl, 2-thiazolyl, or 6-methoxy-2-pyrazinyl; B is phenyl mono- or polysubstituted phenyl [wherein the substituents may be fluoro, alkyl ($C_1$-$C_4$), or trifluoromethyl], benzyl, mono- or disubstituted benzyl [wherein the substituents may be chloro, nitro, or alkyl ($C_1$-$C_3$)], alkyl ($C_1$-$C_7$), cycloalkyl ($C_3$-$C_6$), pyridyl, 2-phenoxypropionyl, alkyl($C_1$-$C_4$)amino, polyfluoro($F_7$-$F_{14}$)polyalkyl($C_3$-$C_7$), mono- or disubstituted phenylamino (wherein the substituents may be chloro, carboxy, or carboethoxy), 6-p-chlorophenylhexyl, 5-(p-chlorobenzoyl)butyl, 2-p-chlorophenoxy-2-isopropyll, or 1-methylbenzyl; R is hydrogen or alkyl ($C_1$-$C_3$); and n is zero; and when X is sulfur: A is pyrazinyl, quinolyl, or methoxypyrazinyl; B is alkyl ($C_1$-$C_4$), cycloalkyl, benzyl, phenethyl, phenyl, or phenyl substituted with at least one substituent [wherein the substituents may be alkyl ($C_1$-$C_4$), alkoxy ($C_1$-$C_4$), halo, carboalkoxy ($C_1$-$C_4$), carboxy, or cyano]; R is hydrogen; and n is one; in association with a pharmaceutically-acceptable carrier.

7. A process for the preparation of a compound selected from those of the formula:

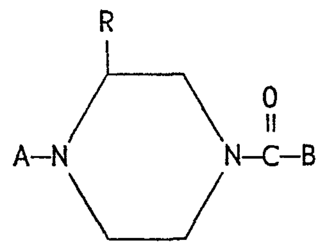

wherein A is phenyl, 2-pyrazinyl, 2-quinoxalinyl, 2-quinolyl, p-chlorophenyl, 3-pyridylmethyl, 2-thiazolyl, or 6-methoxy-2-pyrazinyl; B is phenyl, mono- or polysubstituted phenyl [wherein the substituents may be fluoro, alkyl ($C_1$-$C_4$), or trifluoromethyl], benzyl, mono- or disubstituted benzyl [wherein the substituents may be chloro, nitro, or alkyl ($C_1$-$C_3$)], alkyl ($C_1$-$C_7$), cycloalkyl ($C_3$-$C_6$), pyridyl, 2-phenoxypropionyl, alkyl($C_1$-$C_4$)amino, polyfluoro($F_7$-$F_{14}$)polyalkyl($C_3$-$C_7$), mono- or disubstituted phenylamino (wherein the substituents may be chloro, carboxy, or carboethoxy), 6-p-chlorophenylhexyl, 5-(p-chlorobenzoyl)-butyl, 2-p-chlorophenoxy-2-isopropyl, or 1-methylbenzyl; and R is hydrogen or alkyl ($C_1$-$C_3$), which comprises reacting a substituted phenylacetyl derivative with thionyl chloride and reacting the reaction product so obtained with 1-(2-pyrazinyl)piperazine; or reacting a 2-(1-piperazinyl)aryl derivative with phenylacetyl chloride or reacting a substituted phenylisocyanate with 1-(2-pyrazinyl)-piperazine.

8. A process for preparing a compound of the formula:

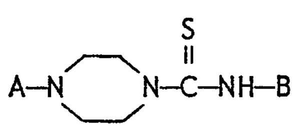

wherein A is pyrazinyl, quinolyl, or methoxypyrazinyl and B is cycloalkyl, benzyl, phenethyl, or substituted phenyl [wherein the substituents may be alkoxy ($C_1$-$C_4$), carboxy, or cyano]; which comprises reacting a piperazine of:

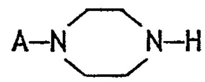

wherein A is as defined above, with an isothiocyanate of the formula:

$S=C=N-B$

wherein B is as defined above, to yield a compound of the formula:

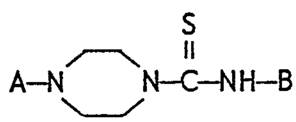

wherein A and B are as defined above.